# EUROPEAN PATENT SPECIFICATION

(11) **EP 4 296 868 B1**
(45) Date of publication and mention of the grant of the patent: **03.07.2024**
(21) Application number: 22180498.2
(22) Date of filing: 22.06.2022
(51) Int. Cl.: G06F 16/65

(54) **METHOD FOR SNORE ATTRIBUTION**
VERFAHREN ZUR SCHNARCHZUORDNUNG
PROCÉDÉ D'ATTRIBUTION DE RONFLEMENT

(43) Date of publication of application: 27.12.2023
(73) Proprietor: Sleep Cycle AB, 41250 Göteborg (SE)
(72) Inventor: VON HOLST, Mikael, Hönö (SE); KÅGEBÄCK, Mikael, Västra Frölunda (SE); LARSSON, Maria, Göteborg (SE)
(74) Representative: AWA Sweden AB

(56) References cited:
- US-A1- 2021 325 521
- ANSARI MOHAMMAD WASIF ET AL: "A Deep Learning Model to Snore Detection Using Smart Phone", 2021 12TH INTERNATIONAL CONFERENCE ON COMPUTING COMMUNICATION AND NETWORKING TECHNOLOGIES (ICCCNT), IEEE, 6 July 2021 (2021-07-06), pages 1-5, XP034009851, DOI: 10.1109/ICCCNT51525.2021.9580153 [retrieved on 2021-10-18]

## Description

### Technical field

The present invention relates to a computer-implemented method for snore attribution. The present invention also relates to a corresponding computer program product and device.

### Background

When several people sleep in the same room and snoring is detected, it is desirable to identify who is snoring.

To this end, CN110349587A discloses to a method for distinguishing snores of target individuals in a two-person scene. The distinguishing method comprises the following three steps: (1) performing target sampling: sampling snore audios of the target individuals in a single-person scene and extracting characteristics for storage. (2) performing sampling in the two-person scene: sampling the snore audios in the two-person scene and extracting characteristics for storage; and (3) performing target individual recognition: distinguishing the snores of the two persons in the two-person scene according to the snore sampling characteristics of the target individuals in the single-person scene, and performing recognition. According to CN110349587A, different individuals can thus be distinguished on the premise of ensuring high-precision snore recognition; and the method is particularly suitable for a sleep monitoring system in the two-person scene.

US 2021/325521 A1 discloses voice attribution to persons using a trained Al model to classify captured voices and includes user-provided attribution.

### Summary of the invention

It is an object to provide an improved method for snore attribution. The invention is defined by the attached claims. According to a first aspect of the present invention, this and other objects are achieved by a computer-implemented method for snore attribution, comprising: capturing audio using a microphone; detecting a plurality of snores in the captured audio; determining that a first set of snores of said plurality of snores belongs to a first individual and that a second set of snores of said plurality of snores belongs to a second individual using a trained model; playing for a user a subset of the snores of said first set and a subset of the snores of said second set; for each played snore, prompting the user via a user interface to provide input whether or not the played snore belongs to the user; receiving said input from the user via the user interface; and attributing the first set of snores or the second set of snores to the user based on said input.

The present invention is at least partly based on the understanding that a trained model can determine that some detected snores in captured audio belongs to one individual and other detected snores in the captured audio belong to another individual. In other words, the trained model may accurately disentangle the snores of different people.

The present invention is also based on the understanding that by letting a user label some of the snores via a user interface (UI), it is possible to determined which snores in the captured audio that belongs to the user. In other words, it is possible to attribute each snore to the right person, even when several individuals are sleeping in the same room. This may in turn allow the user to get deeper knowledge of his (her) snoring.

Furthermore, an advantage of the present method over the distinguishing method in CN1 10349587A is that step (1) performing target sampling: sampling snore audios of the target individuals in a single-person scene and extracting characteristics for storage does not need to be performed. That is, the present method does no need to know anything snoring-related about the user beforehand. It does not even need to know beforehand if there is one or two individuals.

The first and second individuals and the user will typically be persons, but one of the individuals (not the user) could alternatively be a pet.

The method may further comprise attributing the other of the first and second sets of snores to another user. In this way, the method can attribute different snores different persons, even if it only uses one device/smartphone.

Accordingly, the method may be performed on a single device, such as a smartphone, comprising said microphone. Performing the method on the device/smartphone may also be beneficial from an integrity point of view. Thai is, the captured sounds are analysed locally on the device, and not in the

The audio will typically be captured when the first individual and the second individual are (sleeping) in the same room.

The user interface may for each played snore specifically prompt the user to provide input whether the played snore belongs to the user, does not belong to the user, or optionally belongs to multiple individuals or is inaudible. That the played snore does not belong to the user could be expressed explicitly (e.g. 'Not me') or implicitly (e.g. 'Other user').

The user interface may be a graphical user interface implemented on a touch screen, although other user interfaces such as a voice-based user interface could be used as well, or combinations thereof.

The present method forms a multidimensional vector space embedding of each detected snore, wherein determining that a first set of snores of said plurality of snores belongs to a first individual and that a second set of snores of said plurality of snores belongs to a second individual using a trained model includes: the trained model causing the embeddings of the snores of the first individual to be located in a first subspace in a vector space and the embeddings of the snores of the second individual to be located in a second, different subspace in said vector space; clustering the embeddings located in the first subspace to form said first set of snores; and clustering the embeddings located in the second subspace to form said second set of snores.

The trained model may be based on the triplet loss function, which is previously known per se.

During training of the model, two embeddings (anchor and positive) of snores known to belong to the same person are moved closer together in a vector space and another embedding (negative) of a snore known to belong to a different person is moved further away from one of said two embeddings (the anchor). Training the model in this way may constitute a separate aspect of this invention.

Training data for the trained model may include multiple sleep audio clips annotated as snores by multiple users (e.g. >500000 users). That is, each of the multiple users annotates one or more of his/her sleep audio clips as containing a snore. This way of generating training data for use in training the model may constitute a separate aspect of this invention.

According to a second aspect of the invention, there is provided a computer program product comprising computer program code to perform, when executed on a computer, the method according to the first aspect. The computer program product may be a non-transitory computer program product. The computer program product may for example be an app. The computer may be the aforementioned device/smartphone.

According to a third aspect of the invention, there is provided a computer-readable storage medium comprising the computer program product according to the second aspect.

According to a fourth aspect of the invention, there is provided an electrical signal embodied on a carrier wave and propagated on an electrical medium, the electrical signal comprising the computer program product according to the second aspect.

According to a fifth aspect of the invention, there is provided a device comprising a microphone, a processor, audio playback functionality, and a user interface, wherein the device is configured to: capture audio using the microphone; detect, using the processor, a plurality of snores in the captured audio; determine, using the processor and a trained model, that a first set of snores of said plurality of snores belongs to a first individual and that a second set of snores of said plurality of snores belongs to a second individual; play for a user a subset of the snores of said first set and a subset of the snores of said second set using the audio playback functionality; for each played snore, prompt the user via the user interface to provide input whether (or not) the played snore belongs to the user; receive said input from the user (via the user interface); and attribute, using the processor, the first set of snores or the second set of snores to the user based on said input. This aspect of the invention may exhibit the same or similar features and technical effects as any one of the previous aspects, and vice versa.

### Brief description of the drawings

These and other aspects of the present invention will now be described in more detail, with reference to the appended drawings showing currently preferred embodiments of the invention.
Fig. 1 is a schematic view of a device according to an aspect of the present invention, in a two-person scene.
Fig. 2 is a flow chart of a snore attribution method according to another aspect of the present invention.
Fig. 3 is a machine learning flow chart for training a model.
Figs. 4a-b illustrate snore embeddings in a vector space.
Figs. 5a-d illustrate a graphical user interface according to one or more embodiments of the present invention.

### Detailed description of the invention

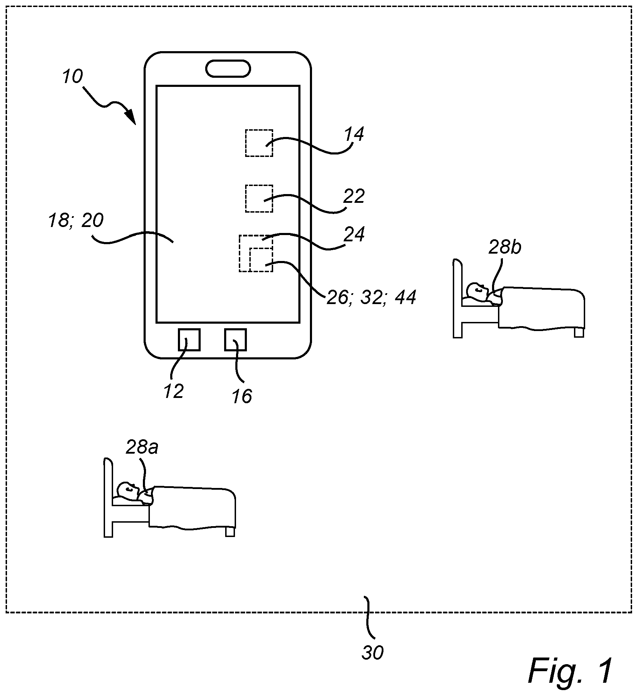

Fig. 1 illustrates a device 10 according to an aspect of the present invention. The device 10 may specifically be mobile or handheld or portable computing device 10. In fig. 1, the device 10 is a smartphone, like an iPhone or an Android phone. Alternatively, the device 10 could be a (regular) mobile phone, a smartwatch or other wearable, a tablet computer, a smart speaker, etc.

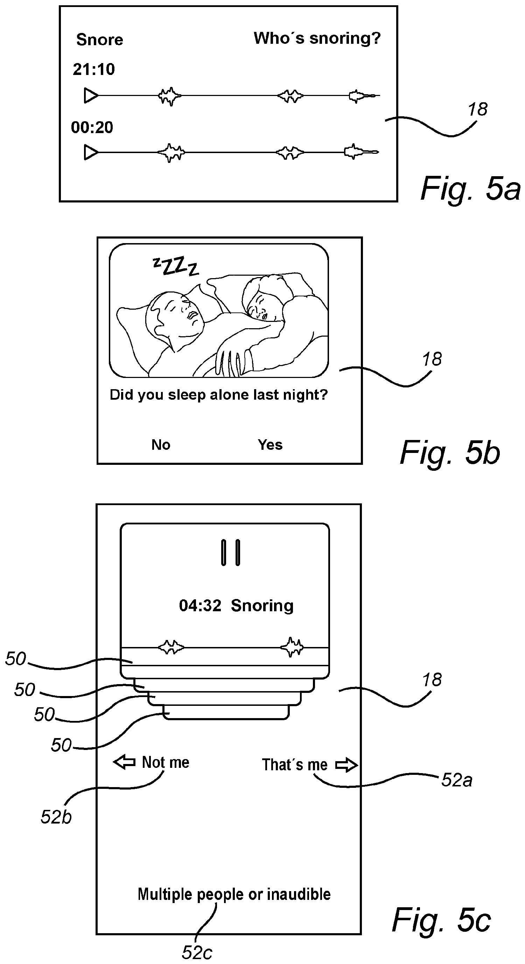

The device 10 may comprise at least one microphone 12 adapted to capture audio, at least one processor 14, audio playback functionality 16, and a user interface 18 (see figs. 5a-c). The user interface 18 may be a graphical user interface implemented on a touch screen 20 of the device 10. The audio playback functionality 16 could be at least one built-in speaker of the device 10 or at least one external speaker, such as wired or wireless headphones. The device 10 may also comprise a memory 22 and a storage 24.

The device 10 may be configured to perform various specific steps or actions detailed in the following, preferably by means of a computer program product 26, for example an app. The computer program product 26 may be downloaded to the device 10 and stored on the aforementioned storage 24. The computer program product 26 may run or be executed on the device 10 using the aforementioned processor 14 and memory 22.

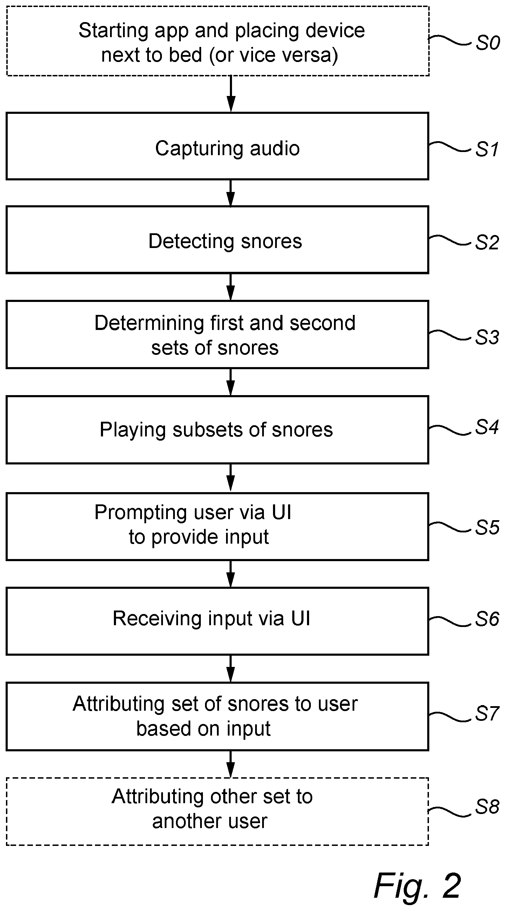

Turning to fig. 2, fig. 2 is a flow chart of a (computer-implemented) method for snore attribution according to one or more embodiments of the present invention.

The method will typically be initiated at S0 by a user starting the computer program product/app 26 and placing the device 10 next to the bed of the user, for example on a nightstand. The user is in fig. 1 one of a first individual 28a and a second individual 28b sleeping in the same room 30.

At S1, the method comprises capturing audio (or sounds) by means of the microphone 12 of the device 10. The audio may be captured over a period of time, including when both individuals 28a-b are sleeping in the room 30. The period of time may for example correspond to the normal length of a night's sleep for humans. As such, the period of time could be in the range of 6 to 10 hours.

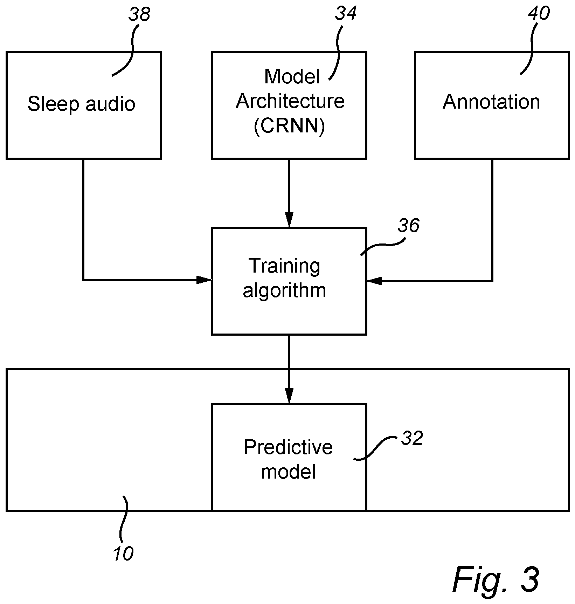

At S2, the method comprises detecting a plurality of snores in the captured audio. The plurality of snores may be detected using a first trained model 32 on the device 10. With further reference to fig. 3, starting from a model architecture 34 (e.g. Convolutional Recurrent Neural Network, CRNN), a training algorithm 36 uses previously recorded sleep audio 38 and corresponding manual annotations (sleep-talking, snoring, etc.) 40, resulting in the first trained model 32. Currently, applicant's first model 32 is trained by approximately 13000 hours of sleep audio and about 4.9 million annotations.

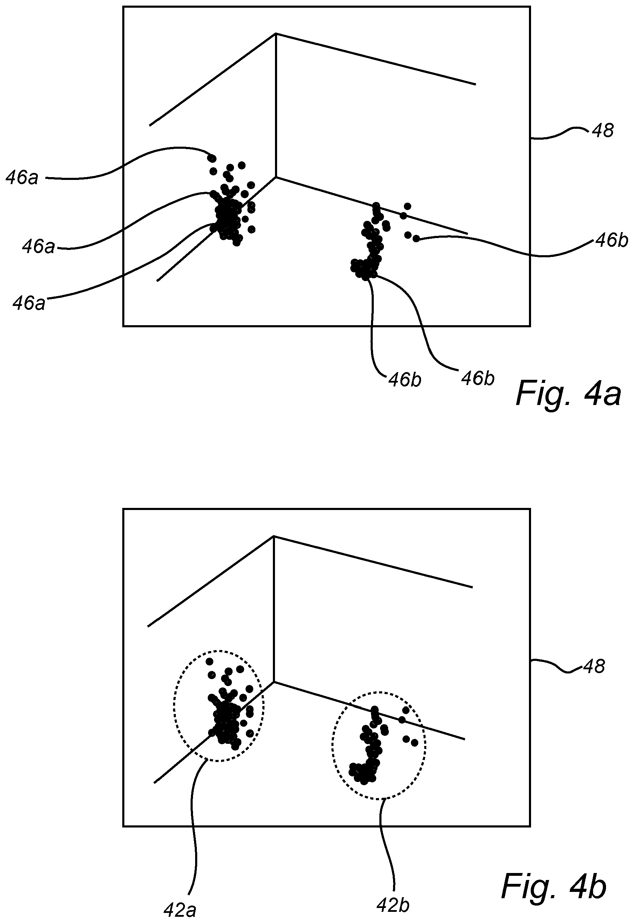

At S3, the method determines that a first set of snores 42a of the plurality of detected snores belongs to the first individual 28a and that a second, different set of snores 42b of the plurality of detected snores belongs to the second individual 28b by means of a second trained model 44 on the device 10. Training data for the second model 44 may include multiple sleep audio clips annotated as snores by multiple users, in the present case more than 500000 users. In other words, the second model 44 has been trained using sleep audio clips annotated as snores from more than 500000 users. In yet other words, the second trained model 44 may be based upon a collected dataset containing more than 500k unique subjects snoring. With further reference to figs. 4a-b, each point in figs. 4a-b is one of the detected snores. Specifically, each point is a high-dimensional vector space embedding 46a-b of a detected snore, here projected down to 3D. The second trained model 44 causes the embeddings 46a of the snores of the first individual 28a to be located in a first subspace in a vector space 48 and the embeddings 46b of the snores of the second individual 28b to be located in a second, different subspace in the vector space 48. The method may then cluster the embeddings 46a located in the first subspace to form the aforementioned first set of snores 42a, and cluster the embeddings 46b located in the second subspace to form the aforementioned second set of snores 42b, as illustrated by the dashed lines in fig. 4b.

The second trained model 44 may be based on the triplet loss function, where during training/learning two embeddings (snores) known to belong to the same person are moved closer together and one embedding known to belong to another person is moved further away in the vector space. In other words, the distance between the two embeddings/snores (anchor and positive) known to belong to the same person is minimized, and the distance from one of those two embeddings/snores (anchor) to the embedding/snore (negative) known to belong to the other person is maximized (up to a certain distance). To this end, the second trained model 44 may in step S3 embed in the way that the embeddings 46a of the snores of the first individual 28a are closer together in the vector space 48 and such that the embeddings 46b of the snores of the second individual 28b are further away from the embeddings 46a in the vector space 48, as illustrated in figs. 4a-b. The training of the second model 44 may be performed by means of a training algorithm, typically off the device 10, similar to the machine learning flow chart of fig. 3.

It should be noted that in case of a one-person scene (i.e. only one individual sleeping in the room 30), the second trained model 44 will place all the embeddings/snores in the same subspace in the vector space 48.

It should also be noted that at this point, the method has determined that two individuals 28a-b have been snoring, but it does not know who is who, in particular which one of the two individuals 28a-b is the aforementioned user? (see fig. 5a) But this can be determined by means of the aforementioned user interface 18. With further reference to fig. 5b, the user may be asked by the user interface 18 if he/she slept alone last night? If the user inputs `Yes' (i.e. in case of a one-person scene as mentioned above), the method will assume that all detected snores belong to the user.

If the user inputs 'No', the method may proceed to S4, where the method includes playing for the user a (first) subset of the snores of the first set 42a and a (second) subset of the snores of the second set 42b using the playback functionality 16. Each subset may for example be 2-5 snores or about 1 % of the total number of snores of the respective set 42a-b. The subsets may for example be the embeddings 46a-b specifically marked in fig. 4a. The snores of the subsets to be played for the user could be randomly selected from the sets 42a-b. Alternatively the snores of the subsets could be chosen such that one snore is closed to the centroid of the respective set 42a-b and the rest are random. Other ways of purposely selecting the snores for the subsets to be played for the user could be used as well.

At S5, for each played snore as illustrated by 50, the method includes prompting the user via the user interface 18 to provide input whether or not the played snore belongs to the user, as shown in fig. 5c. Specifically, the user interface 18 may for each played snore 50 prompt the user to provide input whether the played snore belongs to the user 52a, does not belong to the user 52b, or belongs to multiple individuals or is inaudible or is not a snore at all 52c. And for each played snore, the user may provide the input by pressing/touching/swiping the appropriate button or area 52a-c in the user interface 18 on the touchscreen 20. Here it can be noted that although the user may not always be able to identify his/her own snores, the user is typically able to identity his/her partner's or pet's snores, whoever else is sleeping in the room 30 along with the user. So for played snores that the user does not identify as belonging to the other individual in the room 30 (or that the user identifies as belonging to himself/herself), the user should input that the played snores belong to the user, e.g. by pressing 'That's me' 52a or swiping right. And for played snores that the user does identify as belonging to the other individual in the room 30, the user should input that the played snores do not belong to the user, e.g. by pressing 'Not me' 52b or swiping left.

At S6, the method includes receiving the input(s) from the user via the user interface 18.

And at S7, the method includes attributing either the first set of snores 42a or the second set of snores 42b to the user based on the input received in step S6. For example, if the user indicated that all played snores of the subset of the first set of snores 42a belong to the user (but none of the played snores of the subset of the second set of snores 42b), the method will attribute all (remaining) snores of the first set of snores 42a (but not the snores of the second set 42b) to the user, even if the user has not explicitly confirmed these remaining snores. That is, the user is in this case identified as being the first individual 28a. This may in turn allow the user to get deeper knowledge of his/her snoring, since his/her snoring data for subsequent analysis and/or presentation can exclude snores of the second individual 28b in the same room 30. The analysis and/or presentation may for example include the user's total snoring time, a sleep cycle diagram with the user's snores throughout the night indicated, etc.

The method could also include attributing the other of the first and second sets of snores to another user (step S8). Continuing the above example, all snores of the second set of snores 42b could be attributed to the other user. That is, the other user is in this case identified as being the second individual 28b. In this way, the method can attribute each snore to the correct person 28a-b, even if it only uses one device/smartphone 10. Here, the label 52b could be 'Other user' or similar rather than 'Not me'.

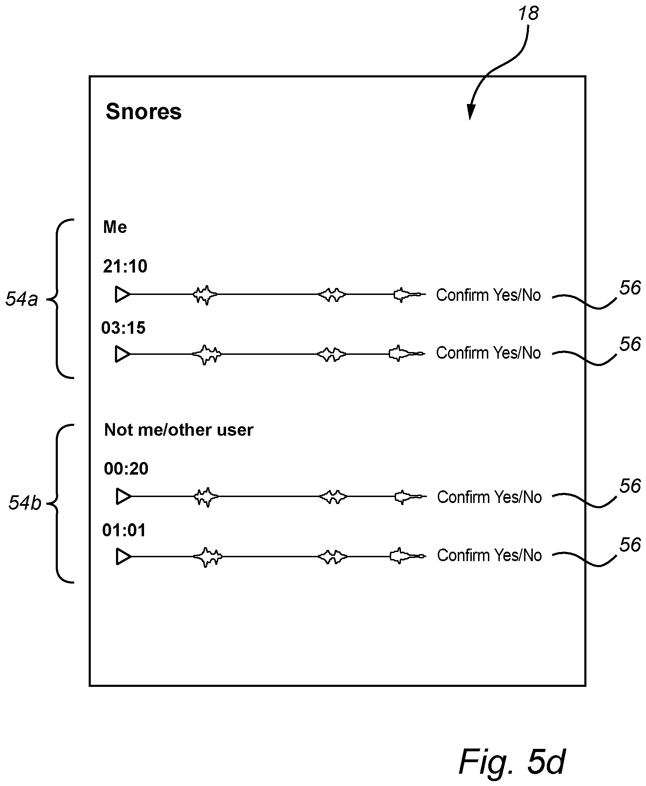

For subsequent periods of times, for example the following nights, the steps S1-S7 could be repeated. Alternatively, only steps S1-S3 (or S0-S3) need to be performed, whereafter the method automatically attributes either the first set of snores or the second set of snores of the subsequent period of time to the user based on the input from at least one previous run of the method (steps S1-S7), without the user having to do anything more. Nevertheless, with further reference to fig. 5d, the method could here prompt/allow the user via the user interface 18 to listen to some of the snores attributed to the user (at 54a) and/or some of snores not attributed to the user (at 54b), and also prompt the user to confirm whether or not these snores have been correctly attributed (at 56). The second model 44 may then be further trained (on the device 10) based on the input from the user whether or not these snores have been correctly attributed. In other words, the user may correct any erroneous attributions, to further train the second model 44.

The person skilled in the art realizes that the present invention by no means is limited to the embodiments described above. On the contrary, many modifications and variations are possible within the scope of the appended claims. For example, the second trained model 44 could be used to determine more than two different sets of snores from the captured audio in case more than two individuals are sleeping (and snoring) in the same room for a period of time, and the steps S4-S8 may be modified accordingly.

## Claims

1. A computer-implemented method for snore attribution, comprising:
capturing audio using a microphone (12);
detecting a plurality of snores in the captured audio;
determining that a first set of snores (42a) of said plurality of snores belongs to a first individual (28a) and that a second set of snores (42b) of said plurality of snores belongs to a second individual (28b) using a trained model (44);
playing for a user a subset of the snores of said first set (42a) and a subset of the snores of said second set (42b);
for each played snore, prompting the user via a user interface (18) to provide input whether or not the played snore belongs to the user;
receiving said input from the user via the user interface; and
attributing the first set of snores or the second set of snores to the user based on said input,
wherein a multidimensional vector space embedding of each detected snore is formed, and wherein determining that a first set of snores of said plurality of snores belongs to a first individual and that a second set of snores of said plurality of snores belongs to a second individual using a trained model includes:
- the trained model (44) causing the embeddings (46a) of the snores of the first individual (28a) to be located in a first subspace in a vector space (48) and the embeddings (46b) of the snores of the second individual to be located in a second, different subspace in said vector space (48);
- clustering the embeddings (46a) located in the first subspace to form said first set of snores (42a); and
- clustering the embeddings (66b) located in the second subspace to form said second set of snores (42b),
wherein the method does not know anything snoring-related about the user beforehand.

2. A computer-implemented method according to claim 1, further comprising attributing the other of the first and second sets of snores to another user.

3. A computer-implemented method according to claim 1 or 2, performed on a single device (10), such as a smartphone, comprising said microphone.

4. A computer-implemented method according to any one of the preceding claims, wherein the audio is captured when the first individual (28a) and the second individual (28b) are in the same room (30).

5. A computer-implemented method according to any one of the preceding claims, wherein the user interface for each played snore prompts the user to provide input whether the played snore belongs to the user (52a), does not belong to the user (52b), or belongs to multiple individuals or is inaudible (52c).

6. A computer-implemented method according to any one of the preceding claims, wherein the user interface is a graphical user interface implemented on a touch screen (20).

7. A computer-implemented method according to claim 1, wherein during training of the trained model (44) two embeddings of snores known to belong to the same person are moved closer together in a vector space and another embedding of a snore known to belong to a different person is moved further away from one of said two embeddings .

8. A computer-implemented method according to any one of the preceding claims, wherein the trained model (44) is based on a triplet loss function.

9. A computer-implemented method according to any one of the preceding claims, wherein training data for the trained model (44) include multiple sleep audio clips annotated as snores by multiple users.

10. A computer program product (26) comprising computer program code to perform, when executed on a computer (10), the method according to any one of the preceding claims.

11. A computer-readable storage medium comprising the computer program product according to claim 10.

12. An electrical signal embodied on a carrier wave and propagated on an electrical medium, the electrical signal comprising the computer program product according to claim 10.

13. A device (10) comprising a microphone (12), a processor (14), audio playback functionality (16), and a user interface (18), wherein the device is configured to:
capture audio using the microphone (12);
detect, using the processor (14), a plurality of snores in the captured audio;
determine, using the processor (14) and a trained model (44), that a first set of snores (42a) of said plurality of snores belongs to a first individual (28a) and that a second set of snores (42b) of said plurality of snores belongs to a second individual (28b), wherein the device is configured to form a multidimensional vector space embedding of each detected snore, the trained model (44) causing the embeddings (46a) of the snores of the first individual (28a) to be located in a first subspace in a vector space (48) and the embeddings (46b) of the snores of the second individual to be located in a second, different subspace in said vector space (48), wherein the device is configured to cluster the embeddings (46a) located in the first subspace to form said first set of snores (42a), and wherein the device is configured to clustering the embeddings (66b) located in the second subspace to form said second set of snores (42b);
play for a user a subset of the snores of said first set (42a) and a subset of the snores of said second set (42b) using the audio playback functionality (16);
for each played snore, prompt the user via the user interface (18) to provide input whether or not the played snore belongs to the user;
receive said input from the user via the user interface; and
attribute, using the processor (14), the first set of snores or the second set of snores to the user based on said input,
wherein the device does not know anything snoring-related about the user beforehand.

## Patentansprüche

1. Computerimplementiertes Verfahren zur Schnarchzuordnung, das Folgendes umfasst:
Aufnehmen von Audio unter Verwendung eines Mikrofons (12) ;
Detektieren einer Mehrzahl von Schnarchtönen in den aufgenommenen Audios;
Bestimmen, dass ein erster Satz von Schnarchtönen (42a) der Mehrzahl von Schnarchtönen zu einem ersten Individuum (28a) gehört und dass ein zweiter Satz von Schnarchtönen (42b) der Mehrzahl von Schnarchtönen zu einem zweiten Individuum (28b) gehört, unter Verwendung eines trainierten Modells (44);
Abspielen einer Teilmenge der Schnarchtöne des ersten Satzes (42a) und einer Teilmenge der Schnarchtöne des zweiten Satzes (42b) für einen Benutzer;
für jeden abgespielten Schnarchton, Auffordern des Benutzers, über eine Benutzerschnittstelle (18) eine Eingabe bereitzustellen, ob der abgespielte Schnarchton zu dem Benutzer gehört oder nicht;
Empfangen der Eingabe von dem Benutzer über die Benutzerschnittstelle; und
Zuordnen des ersten Satzes von Schnarchtönen oder des zweiten Satzes von Schnarchtönen zu dem Benutzer basierend auf dieser Eingabe,
wobei eine mehrdimensionale Vektorraumeinbettung jedes detektierten Schnarchtons gebildet wird, und wobei das Bestimmen, dass ein erster Satz von Schnarchtönen der Mehrzahl von Schnarchtönen zu einem ersten Individuum gehört und dass ein zweiter Satz von Schnarchtönen der Mehrzahl von Schnarchtönen zu einem zweiten Individuum gehört, unter Verwendung eines trainierten Modells beinhaltet:
- das trainierte Modell (44), dass das Einbetten (46a) der Schnarchtöne des ersten Individuums (28a) veranlasst, in einem ersten Unterraum in einem Vektorraum (48) positioniert zu werden, und dass das Einbetten (46b) der Schnarchtöne des zweiten Individuums in einem zweiten, verschiedenen Unterraum in diesem Vektorraum (48) positioniert wird;
- Clustern der Einbettungen (46a), die in dem ersten Unterraum positioniert sind, um den ersten Satz von Schnarchtönen (42a) zu bilden; und
- Clustern der Einbettungen (66b), die in dem zweiten Unterraum positioniert sind, um den zweiten Satz von Schnarchtönen (42b) zu bilden;
wobei das Verfahren im Voraus keine schnarchbezogenen Informationen über den Benutzer kennt.

2. Computerimplementiertes Verfahren nach Anspruch 1, das ferner das Zuordnen des anderen des ersten und des zweiten Satzes von Schnarchtönen zu einem anderen Benutzer umfasst.

3. Computerimplementiertes Verfahren nach Anspruch 1 oder 2, das auf einer einzigen Vorrichtung (10), wie beispielsweise einem Smartphone, durchgeführt wird, das das Mikrofon umfasst.

4. Computerimplementiertes Verfahren nach einem der vorhergehenden Ansprüche, wobei das Audio aufgenommen wird, wenn sich das erste Individuum (28a) und das zweite Individuum (28b) in demselben Raum (30) befinden.

5. Computerimplementiertes Verfahren nach einem der vorhergehenden Ansprüche, wobei die Benutzerschnittstelle für jeden abgespielten Schnarchton den Benutzer auffordert, eine Eingabe bereitzustellen, ob der abgespielte Schnarchton zu dem Benutzer (52a) gehört, nicht zu dem Benutzer (52b) gehört oder zu mehreren Individuen gehört oder unhörbar (52c) ist.

6. Computerimplementiertes Verfahren nach einem der vorhergehenden Ansprüche, wobei die Benutzerschnittstelle eine auf einem Touchscreen (20) implementierte grafische Benutzerschnittstelle ist.

7. Computerimplementiertes Verfahren nach Anspruch 1, wobei während des Trainings des trainierten Modells (44) zwei Einbettungen von Schnarchtönen, von denen bekannt ist, dass sie zu derselben Person gehören, in einem Vektorraum näher zusammengerückt werden und eine andere Einbettung eines Schnarchtons, von dem bekannt ist, dass es zu einer verschiedenen Person gehört, weiter von einer der beiden Einbettungen weggerückt wird.

8. Computerimplementiertes Verfahren nach einem der vorhergehenden Ansprüche, wobei das trainierte Modell (44) auf einer Triplett-Verlustfunktion basiert.

9. Computerimplementiertes Verfahren nach einem der vorhergehenden Ansprüche, wobei die Trainingsdaten für das trainierte Modell (44) mehrere Schlaf-Audioclips beinhalten, die von mehreren Benutzern als Schnarchtöne annotiert wurden.

10. Computerprogrammprodukt (26), das Computerprogrammcode umfasst, um, wenn auf einem Computer (10) ausgeführt, das Verfahren nach einem der vorhergehenden Ansprüche durchzuführen.

11. Computerlesbares Speichermedium, das das Computerprogrammprodukt nach Anspruch 10 umfasst.

12. Elektrisches Signal, das auf einer Trägerwelle verkörpert ist und sich auf einem elektrischen Medium ausbreitet, wobei das elektrische Signal das Computerprogrammprodukt nach Anspruch 10 umfasst.

13. Vorrichtung (10), die ein Mikrofon (12), einen Prozessor (14), eine Audiowiedergabefunktionalität (16) und eine Benutzerschnittstelle (18) umfasst, wobei die Vorrichtung dazu ausgelegt ist:
Audio unter Verwendung des Mikrofons (12) aufzunehmen;
unter Verwendung des Prozessors (14), eine Mehrzahl von Schnarchtönen in den aufgenommenen Audios zu detektieren;
unter Verwendung des Prozessors (14) und eines trainierten Modells (44) zu bestimmen, dass ein erster Satz von Schnarchtönen (42a) der Mehrzahl von Schnarchtönen zu einem ersten Individuum (28a) gehört und dass ein zweiter Satz von Schnarchtönen (42b) der Mehrzahl von Schnarchtönen zu einem zweiten Individuum (28b) gehört, wobei die Vorrichtung dazu ausgelegt ist, eine mehrdimensionale Vektorraumeinbettung jedes detektierten Schnarchtons zu bilden, wobei das trainierte Modell (44) die Einbettungen (46a) der Schnarchtöne des ersten Individuums (28a) veranlasst, in einem ersten Unterraum in einem Vektorraum (48) positioniert zu werden und die Einbettungen (46b) der Schnarchtöne des zweiten Individuums in einem zweiten, verschiedenen Unterraum in diesem Vektorraum (48) positioniert zu werden, wobei die Vorrichtung dazu ausgelegt ist, die im ersten Unterraum befindlichen Einbettungen (46a) zu clustern, um den ersten Satz von Schnarchtönen (42a) zu bilden, und wobei die Vorrichtung dazu ausgelegt ist, die im zweiten Unterraum befindlichen Einbettungen (66b) zu clustern, um den zweiten Satz von Schnarchtönen (42b) zu bilden;
für einen Benutzer eine Teilmenge der Schnarchtöne des ersten Satzes (42a) und eine Teilmenge der Schnarchtöne des zweiten Satzes (42b) unter Verwendung der Audiowiedergabefunktionalität (16) abzuspielen;
für jeden abgespielten Schnarchton den Benutzer über die Benutzerschnittstelle (18) aufzufordern, eine Eingabe bereitzustellen, ob der abgespielte Schnarchton dem Benutzer gehört oder nicht;
die Eingabe von dem Benutzer über die Benutzerschnittstelle zu empfangen; und
unter Verwendung des Prozessors (14), den ersten Satz von Schnarchtönen oder den zweiten Satz von Schnarchtönen dem Benutzer basierend auf dieser Eingabe zuzuordnen,
wobei die Vorrichtung im Voraus keine schnarchbezogenen Informationen über den Benutzer kennt.

## Revendications

1. Procédé d'attribution de ronflements mis en œuvre par ordinateur, comprenant les étapes suivantes :
capture d'un signal audio au moyen d'un microphone (12) ;
détection d'une pluralité de ronflements dans le signal audio capturé ;
détermination du fait qu'un premier ensemble de ronflements (41a) de ladite pluralité de ronflements appartient à un premier individu (28a) et qu'un deuxième ensemble de ronflements (42b) de ladite pluralité de ronflements appartient à un deuxième individu (28b) au moyen d'un modèle entraîné (44) ;
lecture à destination d'un utilisateur d'un sous-ensemble des ronflements dudit premier ensemble (41a) et d'un sous-ensemble de ronflements dudit deuxième ensemble (42b) ;
pour chaque ronflement lu, demande à l'utilisateur via une interface utilisateur (18) de saisir si oui ou non le ronflement lu appartient à l'utilisateur ;
réception de ladite saisie de l'utilisateur via l'interface utilisateur ; et
attribution du premier ensemble de ronflements ou du deuxième ensemble de ronflements à l'utilisateur en fonction de ladite saisie,
une incorporation de chaque ronflement détecté dans un espace vectoriel multidimensionnel étant formée, et la détermination du fait qu'un premier ensemble de ronflements de ladite pluralité de ronflements appartient à un premier individu et qu'un deuxième ensemble de ronflements de ladite pluralité de ronflements appartient à un second individu au moyen d'un modèle entraîné comprenant :
- le placement, par le modèle entraîné (44), des incorporations (46a) des ronflements du premier individu (28a) dans un premier sous-espace d'un espace vectoriel (48) et des incorporations (46b) des ronflements du deuxième individu dans un deuxième sous-espace, différent, dans ledit espace vectoriel (48) ;
- le regroupement des incorporations (46a) placées dans le premier sous-espace de façon à former ledit premier ensemble de ronflements (42a) ; et
- le regroupement des incorporations (66b) placées dans le deuxième sous-espace de façon à former ledit deuxième ensemble de ronflements (42b),
le procédé n'ayant aucune connaissance préalable relative aux ronflements de l'utilisateur.

2. Procédé mis en œuvre par ordinateur selon la revendication 1, comprenant en outre l'attribution de l'autre des premier et deuxième ensembles de ronflements à un autre utilisateur.

3. Procédé mis en œuvre par ordinateur selon la revendication 1 ou 2, exécuté sur un seul dispositif (10), tel qu'un smartphone, comprenant ledit microphone.

4. Procédé mis en œuvre par ordinateur selon l'une quelconque des revendications précédentes, dans lequel le signal audio est capturé lorsque le premier individu (28a) et le deuxième individu (28b) sont dans la même pièce (30).

5. Procédé mis en œuvre par ordinateur selon l'une quelconque des revendications précédentes, dans lequel l'interface utilisateur, pour chaque ronflement lu, demande à l'utilisateur de saisir si le ronflement lu appartient à l'utilisateur (52a), n'appartient pas à l'utilisateur (52b) ou appartient à plusieurs individus ou est inaudible (52c).

6. Procédé mis en œuvre par ordinateur selon l'une quelconque des revendications précédentes, dans lequel l'interface utilisateur est une interface utilisateur graphique mise en œuvre sur un écran tactile (20).

7. Procédé mis en œuvre par ordinateur selon la revendication 1, dans lequel, pendant l'entraînement du modèle entraîné (44), deux incorporations de ronflements connus comme appartenant à la même personne sont rapprochées l'une de l'autre dans un espace vectoriel et une autre incorporation d'un ronflement connu comme appartenant à une personne différente est éloignée de l'une desdites deux incorporations.

8. Procédé mis en œuvre par ordinateur selon l'une quelconque des revendications précédentes, dans lequel le modèle entraîné (44) est basé sur une fonction de coût par triplet.

9. Procédé mis en œuvre par ordinateur selon l'une quelconque des revendications précédentes, dans lequel les données d'entraînement pour le modèle entraîné (44) incluent plusieurs clips audio de sommeil annotés en ce qui concerne les ronflements de plusieurs utilisateurs.

10. Produit de programme informatique (26) comprenant un code de programme informatique pour mettre en oeuvre, lorsqu'il est exécuté sur un ordinateur (10), le procédé selon l'une quelconque des revendications précédentes.

11. Support de stockage lisible par ordinateur comprenant le produit de programme informatique selon la revendication 10.

12. Signal électrique transporté sur une onde porteuse et propagé sur un support électrique, le signal électrique comprenant le produit de programme informatique selon la revendication 10.

13. Dispositif (10) comprenant un microphone (12), un processeur (14), un fonctionnalité de lecture audio (16) et une interface utilisateur (18), lequel dispositif est configuré pour :
capturer un signal audio au moyen du microphone (12) ;
détecter, au moyen du processeur (14), une pluralité de ronflements dans le signal audio capturé ;
déterminer, au moyen du processeur (14) et d'un modèle entraîné (44), qu'un premier ensemble de ronflements (42a) de ladite pluralité de ronflements appartient à un premier individu (28a) et qu'un deuxième ensemble de ronflements (42b) de ladite pluralité de ronflements appartient à un deuxième individu (28b), le dispositif étant configuré pour former une incorporation de chaque ronflement détecté dans un espace vectoriel multidimensionnel, le modèle entraîné (44) plaçant les incorporations (46a) des ronflements du premier individu (28a) dans un premier sous-espace d'un espace vectoriel (48) et les incorporations (46b) des ronflements du deuxième individu dans un deuxième sous-espace, différent, dans ledit espace vectoriel (48), le dispositif étant configuré pour regrouper les incorporations (46a) placées dans le premier sous-espace de façon à former ledit premier ensemble de ronflements (42a), et le dispositif étant configuré pour regrouper les incorporations (66b) placées dans le deuxième sous-espace de façon à former ledit deuxième ensemble de ronflements (42b) ;
lire à destination d'un utilisateur un sous-ensemble des ronflements dudit premier ensemble (41a) et un sous-ensemble de ronflements dudit deuxième ensemble (42b) au moyen de la fonctionnalité de lecture audio (16) ;
pour chaque ronflement lu, demander à l'utilisateur via l'interface utilisateur (18) de saisir si oui ou non le ronflement lu appartient à l'utilisateur ;
recevoir ladite saisie de l'utilisateur via l'interface utilisateur ; et
attribuer, au moyen du processeur (14), le premier ensemble de ronflements ou le deuxième ensemble de ronflements à l'utilisateur en fonction de ladite saisie, le dispositif n'ayant aucune connaissance préalable relative aux ronflements de l'utilisateur.
